# EUROPEAN PATENT SPECIFICATION

(11) **EP 3 897 856 B1**
(45) Date of publication and mention of the grant of the patent: **27.11.2024**
(21) Application number: 18849458.7
(22) Date of filing: 19.12.2018
(51) Int. Cl.: A61Q 1/00, A61Q 17/04, A61K 8/81, A61K 8/06

(54) **OIL-IN-WATER EMULSIFIED COSMETIC WITH ULTRAVIOLET RAY-PROTECTING EFFECT**
KOSMETISCHE ÖL-IN-WASSER-EMULSION MIT UV-STRAHLENSCHUTZ
EMULSION COSMETIQUE DE TYPE HUILE DANS EAU AVEC UNE PROTECTION ANTI UV

(43) Date of publication of application: 27.10.2021
(73) Proprietor: L V M H RECHERCHE, 45800 St. Jean de Braye (FR)
(72) Inventor: KISHIDA, Koichi, Tokyo 102--0092 (JP); OZAWA, Mai, Tokyo 102--0092 (JP); SAKODA, Takayoshi, Tokyo 102--0092 (JP)
(74) Representative: Regimbeau
(86) International application number: PCT/IB2018/001594
(87) International publication number: WO 2020/128556

(56) References cited:
- EP-A1- 2 543 356
- WO-A1-2018/109354
- WO-A1-2018/124287
- JP-A- 2008 162 930
- JP-K1- 2008 162 930
- DATABASE GNPD [online] MINTEL; 22 June 2018 (2018-06-22), ANONYMOUS: "Suncare Milk SPF 50", XP055594562, retrieved from www.gnpd.com Database accession no. 5767235

## Description

### Technical Field

The present invention relates to an oil-in-water emulsified cosmetic having a high ultraviolet ray-protecting effect.

### Background Art

Consumers of ultraviolet absorber-containing sunscreen cosmetics prefer those having a light, fresh sensation, and therefore oil-in-water emulsion type cosmetics are commonly in wide use.

It is usually necessary to add large amounts of ultraviolet absorbers if the goal is to impart a high ultraviolet ray-protecting effect in sunscreen cosmetics, in particular for protection of keratinic materials from sun-damage, sunburn, and/or photo-aging. However, ultraviolet absorbers may create discomfort including stickiness, and may also destabilize the emulsion due to their high polarity. Moreover, highly polar oil agents must be added in order to dissolve highly crystalline ultraviolet absorbers. Ongoing efforts are therefore being made to create sunscreen cosmetics that have reduced ultraviolet absorber contents while maintaining their high ultraviolet ray-protecting effects.

### Technical Problem

One approach that is known for the reducing ultraviolet absorber content of a sunscreen cosmetic while maintaining a high ultraviolet ray-protecting effect is to combine an oil-soluble ultraviolet absorber with a water-soluble ultraviolet absorber. However, water-soluble ultraviolet absorbers tend to lower stability because they are electrolytes that affect the emulsified state. WO2007/122822 and JP2018-8907 disclose examples of such oil-in-water emulsified cosmetics which contain an oil-soluble ultraviolet absorber and a water-soluble ultraviolet absorber with addition of a specific surfactant and higher alcohol to achieve a more stable emulsified state and minimize stickiness due to the ultraviolet absorber, but it then becomes necessary to add a considerable amount of a surfactant, which creates a heavy, creamy sensation and lacks a fresh and watery sensation during application.

JP2008-162930 discloses an oil-in-water emulsified sunscreen cosmetic composition comprising an oil-soluble ultraviolet absorber, a water-soluble thickener, a water- soluble ultraviolet absorber and hydrophilic nonionic surfactant.

So there is still a need to provide oil-in-water emulsified cosmetic having a high ultraviolet ray-protecting effect, but with improved stability and providing a fresh and watery sensation when applied on keratinic materials, in particular the skin.

It is an object of the invention to answer this technical problem. The present inventors have found that if a specific crosspolymer is added to an oil-in-water emulsified cosmetic, it is possible to stably disperse the oil phase (oil-soluble ultraviolet absorber and oil agent) even if the total amount of added surfactant is minimal, and that this provides a fresh and watery sensation during application while maintaining a high ultraviolet ray-protecting effect by the oil-in-water emulsified cosmetic..

### Summary of the invention

So the present invention provides an oil-in-water emulsified cosmetic comprising, in a physiologically acceptable medium, (a) an aqueous phase, (b) an anionic surfactant, (c) an oil-soluble ultraviolet absorber, (d) a water-soluble ultraviolet absorber and (e) a crosspolymer including a (meth)acrylamidealkylsulfonic acid or its salt, a dialkyl (meth)acrylamide and a (meth)acrylic acid ester as structural units, wherein content of component (e) ranges from 0.01 to 1.5 mass % based on the total mass of the oil-in-water emulsified cosmetic. The "oil-in-water emulsified cosmetic" may also be referred to hereunder as "oil-in-water emulsion" or "oil-in-water emulsion composition" or "cosmetic composition in form of and oil-in-water emulsion". The "aqueous phase (a)" may also be referred to hereunder simply as "component (a)", and the other components may be referred to in a similar manner. As used herein, the term "(meth)acryl" refers to acryl or methacryl, and the same applies to analogous backbones.
The "mass%" may also be referred to hereunder as "% by weight".
The term "cosmetically or physiologically acceptable" means compatible with the keratinic materials, in particular the skin, which has a color, a smell and a pleasant touch and does not cause unacceptable discomfort (stinging, tautness).

The present invention also relates to the oil-in-water emulsified cosmetic of the invention for use for preventing and/or reducing the effects of UV radiations onto the keratinic materials, in particular onto the skin, in particular protecting the skin from sun-damage, sunburn, and/or photo-aging.

### Detailed description of the invention

The oil-in-water emulsified cosmetic of the invention may further comprise at least one component selected from the group consisting of (f) nonionic surfactants and (g) higher alcohols of 12 to 24 carbon atoms. By adding component (f) and/or component (g), the stability of the emulsified state of the oil-in-water emulsified cosmetic will be even improved.

In the oil-in-water emulsified cosmetic of the invention, the total content of the anionic surfactant, nonionic surfactant and higher alcohol of 12 to 24 carbon atoms may be from 1 to 5 mass%, preferably from 2 to 4.8 mass% based on the total mass of the oil-in-water emulsified cosmetic. An oil-in-water emulsified cosmetic containing the anionic surfactant, the nonionic surfactant and the higher alcohol of 12 to 24 carbon atoms within this range can provide a more fresh and watery sensation during application.

The oil-in-water emulsified cosmetic of the invention may further comprise (h) methylene bis-benzotriazolyl tetramethylbutylphenol. Adding component (h) further improves the ultraviolet ray-protecting effect.

The oil-in-water emulsified cosmetic of the invention has a high ultraviolet ray-protecting effect and can therefore serve as a sunscreen cosmetic composition.

### Advantageous Effects of Invention

The invention provides an oil-in-water emulsified cosmetic with a high ultraviolet ray-protecting effect and having excellent stability, and providing a fresh and watery sensation when applied on keratinic materials, in particular applied on skin. The oil-in-water emulsified cosmetic of the invention is also advantageous in that it lacks stickiness after application.

Preferred embodiments of the invention will now be described, with the understanding that these embodiments are in no way limitative on the invention.

### Aqueous phase

The oil-in-water emulsified cosmetic of the invention contains an aqueous phase (a). The aqueous phase may consist of water alone, or it may also include a solvent that is soluble in water.

The water used may be distilled water, purified water, hot spring water, deep water, or plant-derived steam distilled water such as lavender water, rose water or orange flower water.

Examples of solvents that are soluble in water include mono-alcohols such as methanol, ethanol, propyl alcohol, isopropyl alcohol and phenoxyethanol; and polyhydric alcohols such as ethylene glycol, 1,3-propanediol, propylene glycol, butylene glycol, pentylene glycol, hexylene glycol, dipropylene glycol, polyethylene glycol, glycerin, diglycerin, polyglycerin, sorbitol, xylitol and mannitol, and mixtures thereof. The solvent that is soluble in water may be a single one used alone, or two or more may be used in combination.

The content of the aqueous phase (a) is preferably 40 to 95 mass% and more preferably 50 to 90 mass% based on the total mass of the oil-in-water emulsion. As used herein, "based on the total mass" means based on the total mass of the oil-in-water emulsified cosmetic.

### Anionic surfactant

The oil-in-water emulsified cosmetic of this embodiment contains an anionic surfactant (b).

Examples of anionic surfactants include carboxylic acid-type anionic surfactants including aliphatic monocarboxylic acid salts (for example, sodium laurate, sodium myristate, sodium palmitate and sodium stearate), polyoxyethylene alkyl ether carboxylates (for example, polyoxyethylene lauryl ether acetates), N-acyl sarcosinates (for example, sodium lauroylsarcosinate and sodium myristoylsarcosinate) and N-acylglutamates (for example, sodium lauroylglutamate and sodium stearoylglutamate); sulfonic acid-type anionic surfactants including dialkylsulfosuccinates (for example, sodium di-2-ethylhexylsulfosuccinate), alkanesulfonates, alpha-olefinsulfonates, alkylbenzenesulfonates, alkylnaphthalenesulfonates and N-methyl-N-acyltaurine salts (for example, lauroyl methyltaurine sodium and myristoyl methyltaurine sodium); sulfuric acid ester-type anionic surfactants including alkylsulfates (for example, sodium lauryl sulfate and sodium myristyl sulfate) and polyoxyethylene alkyl ether sulfates (for example, sodium polyoxyethylene lauryl sulfate); and phosphoric acid ester-type anionic surfactants including alkylphosphates (for example, sodium laurylphosphate and potassium cetylphosphate) and polyoxyethylene alkyl ether phosphates (for example, sodium polyoxyethylene stearyl ether phosphate) and mixtures thereof. Of these, N-acylglutamates such as sodium stearoylglutamate and alkylphosphates such as potassium cetylphosphate are preferred for improved stability of the emulsified state. The anionic surfactant used may be a single type alone, or two or more may be used in combination.

The content of component (b) is preferably from 0.6 to 2 mass% and more preferably from 0.7 to 1.8 mass% based on the total mass of the oil-in-water emulsified cosmetic.

### Oil-soluble ultraviolet absorber

The oil-in-water emulsified cosmetic of this embodiment contains an oil-soluble ultraviolet absorber (c).

Examples of oil-soluble ultraviolet absorbers include cinnamic acid-based ultraviolet absorbers, including cinnamic acid-based ultraviolet absorbers such as 2-ethylhexyl paramethoxycinnamate, 2-ethoxyethyl paramethoxycinnamate, isopropyl paramethoxycinnamate-diisopropylcinnamic acid ester mixtures and methyl bis(trimethylsiloxy)silylisopentyl trimethoxycinnamate; salicylic acid-based ultraviolet absorbers such as amyl paradimethylaminobenzoate, 2-ethylhexyl paradimethylaminobenzoate, ethyleneglycol salicylate, 2-ethylhexyl salicylate, benzyl salicylate and homomenthyl salicylate (homosalate); hexyl diethylaminohydroxybenzoylbenzoate; bisethylhexyloxyphenolmethoxyphenyltriazine; ethylhexyltriazone; 2-ethylhexyl dimethoxybenzylidenedioxoimidazolidinepropionate; and t-butylmethoxydibenzoylmethane, and mixtures thereof. The oil-soluble ultraviolet absorber used may be of a single type or a combination of two or more types, but using a combination of two or more types is preferred to exhibit a higher ultraviolet ray-protecting effect. Specifically, it is preferred to use combinations of two or more ultraviolet absorbers selected from the group consisting of cinnamic acid-based ultraviolet absorbers, salicylic acid-based ultraviolet absorbers, 2-ethylhexyl paramethoxycinnamate, hexyl diethylaminohydroxybenzoylbenzoate and bisethylhexyloxyphenolmethoxyphenyltriazine.

The content of component (c) is preferably from 10 to 20 mass% and more preferably from 11 to 16 mass% based on the total mass of the oil-in-water emulsified cosmetic.

### Water-soluble ultraviolet absorber

The oil-in-water emulsified cosmetic of this embodiment contains a water-soluble ultraviolet absorber (d).

Examples of water-soluble ultraviolet absorbers include hydroxymethoxybenzophenone sulfonic acid, phenylbenzimidazole sulfonic acid, phenyldibenzimidazole tetrasulfonic acid and terephthalylidene dicamphorsulfonic acid and mixtures thereof. Of these, phenylbenzimidazole sulfonic acid and phenyldibenzimidazole tetrasulfonic acid are preferred, and phenylbenzimidazole sulfonic acid is more preferred, for providing excellent biostability and a satisfactory ultraviolet ray-protecting effect.

The content of component (d) is preferably from 0.1 to 5 mass% and more preferably from 0.5 to 3 mass% based on the total mass of the oil-in-water emulsified cosmetic.

### Crosspolymer

The oil-in-water emulsified cosmetic of this embodiment contains a crosspolymer including a (meth)acrylamidealkylsulfonic acid or its salt, a dialkyl (meth)acrylamide and a (meth)acrylic acid ester as structural units (e).

An example of a (meth)acrylamide alkylsulfonic acid as a structural unit is (meth)acrylamide methylpropanesulfonic acid. Salts of (meth)acrylamide alkylsulfonic acids include, for example, alkali metal salts such as sodium salts and potassium salts, and also ammonium salts. Ammonium salt is preferred as a salt of (meth)acrylamide alkylsulfonic acid.

An example of a dialkyl (meth)acrylamide as a structural unit is dimethyl (meth)acrylamide.

Examples of (meth)acrylic acid esters as structural units include alkyl (meth)acrylates and alkylpolyoxyethylene (meth)acrylates.

Examples of alkyl chain lengths for alkyl (meth)acrylates include lengths of 6 to 22 carbon atoms. In the oil-in-water emulsified cosmetic of this embodiment, it is preferred to use an alkyl (meth)acrylate having an alkyl group of 10 to 16 carbon atoms. Preferred among these are lauryl (meth)acrylate, myristyl (meth)acrylate and palmityl (meth)acrylate, with lauryl (meth)acrylate being more preferred.

Examples of alkyl chain lengths for alkylpolyoxyethylene (meth)acrylates include alkyl groups of 6 to 22 carbon atoms. In the oil-in-water emulsified cosmetic of this embodiment, it is preferred to use an alkylpolyoxyethylene (meth)acrylate having an alkyl group of 10 to 16 carbon atoms. Lauryl polyoxyethylene (meth)acrylate, myristyl polyoxyethylene (meth)acrylate and palmityl polyoxyethylene (meth)acrylate are more preferred, and lauryl tetraoxyethylene (meth)acrylate is even more preferred. The average number of moles of addition of ethylene oxide is preferably 1 to 30.

Component (e) may include two or more (meth)acrylic acid esters, and it preferably includes an alkyl (meth)acrylate and an alkylpolyoxyethylene (meth)acrylate.

Preferred for component (e) are (meth)acrylamidemethylpropanesulfonic acid and its salts, and crosspolymers including dimethyl (meth)acrylamide, C₆₋₂₂ alkyl (meth)acrylates and C₆₋₂₂ alkylpolyoxyethylene (meth)acrylates as structural units. A specific example for component (e) is ammonium (meth)acrylamide methylpropanesulfonate/dimethyl (meth)acrylamide/lauryl (meth)acrylate/lauryltetraoxyethylene (meth)acrylate crosspolymer (POLYACRYLATE CROSSPOLYMER-6). POLYACRYLATE CROSSPOLYMER-6 is commercially available as SEPIMAX ZEN (product of Seppic Co.).

The content of component (e) is from 0.01 to 1.5 mass% and more preferably from 0.1 to 1 mass% based on the total mass of the oil-in-water emulsified cosmetic.

### Additional ingredients

The oil-in-water emulsified cosmetic of this embodiment may further contain at least one component selected from the group consisting of (f) nonionic surfactants, (g) higher alcohols of 12 to 24 carbon atoms, and mixtures thereof.

### Nonionic surfactants

Examples of nonionic surfactants include glycerin fatty acid esters (for example, glyceryl monostearate and glyceryl monooleate), sorbitan fatty acid esters (for example, sorbitan monostearate, sorbitan monooleate, sorbitan tristearate and sorbitan trioleate), sucrose fatty acid esters, polyoxyethylene alkyl ethers (for example, polyoxyethylene lauryl ether, polyoxyethylene cetyl ether, polyoxyethylene stearyl ether and polyoxyethylene oleyl ether), polyoxyethylene alkylphenyl ethers, alkyl glucosides (for example, decyl glucoside, lauryl glucoside and cocoglycoside), polyethylene glycols fatty acids (for example, polyethylene glycol monolaurate and polyethylene glycol monostearate) and polyoxyethylene sorbitan fatty acids (for example, polyoxyethylene sorbitan monostearate, polyoxyethylene sorbitan monooleate, polyoxyethylene sorbitan tristearate and polyoxyethylene sorbitan trioleate), and mixtures thereof. Alkyl glucosides are especially preferred among these. The nonionic surfactant used may be a single type alone, or two or more may be used in combination.

### Higher alcohols of 12 to 24 carbon atoms

Examples of higher alcohols of 12 to 24 carbon atoms include straight-chain aliphatic monools such as lauryl alcohol, myristyl alcohol, cetyl alcohol (cetanol), stearyl alcohol, behenyl alcohol and oleyl alcohol; and branched chain aliphatic monools such as lanolin alcohol and isostearyl alcohol, and mixtures thereof. Straight-chain aliphatic monools are preferred. A single higher alcohol of 12 to 24 carbon atoms may be used alone, or two or more may be used in combination.

The total content of component (b), component (f) and component (g) is preferably from 1 to 5 mass% and more preferably from 2 to 4.8 mass% based on the total mass of the oil-in-water emulsified cosmetic. This particular range imparts advantageously a fresh and watery sensation during application.

### Methylene bis-benzotriazolyl tetramethylbutylphenol

The oil-in-water emulsified cosmetic of this embodiment may further contain (h) methylene bis-benzotriazolyl tetramethylbutylphenol.

Methylene bis-benzotriazolyl tetramethylbutylphenol is insoluble in water and may therefore be added by dispersion in the aqueous phase. For example, an aqueous dispersion of methylene bis-benzotriazolyl tetramethylbutylphenol marketed under the trade name of TINOSORB M by BASF Corp. may be used (INCI Name: Methylene Bis-Benzotriazolyl Tetramethylbutylphenol (and) Aqua (and) Decyl Glucoside (and) Propylene Glycol (and) Xanthan Gum). This is an organic UV filter using microfine particle technology and acts as both a micropigment and an UV absorber.

The content of component (h) is preferably from 0.5 to 10 mass% and more preferably from 1 to 5 mass% based on the total mass of the oil-in-water emulsified cosmetic. When an aqueous dispersion of methylene bis-benzotriazolyl tetramethylbutylphenol is used as component (h), its content is preferably from 1 to 20 mass% and more preferably from 2 to 10 mass%, based on the total mass of the oil-in-water emulsified cosmetic.

### Additional gelling or thickening agent

In a particular embodiment, the oil-in-water emulsified cosmetic may further contain a thickening agent other than component (e).

Examples for the thickening agent other than component (e) are polymers including acryloylalkyltaurines or their salts as structural units, such as acryloyldimethyltaurine ammonium/vinylpyrrolidone) copolymer, (hydroxyethyl acrylate/acryloyldimethyltaurine sodium) copolymer, (sodium acrylate/acryloyldimethyltaurine sodium) copolymer and (acryloyldimethyltaurine ammonium/ Beheneth-25 methacrylate) crosspolymer; and xanthan gum and mixtures thereof. Preferred among these are (acryloyldimethyltaurine ammonium/vinylpyrrolidone) copolymer and (hydroxyethyl acrylate/acryloyldimethyltaurine sodium) copolymer, since adding such components as thickening agents other than component (e) can impart a more fresh and watery sensation during application.

In a particular embodiment, the oil-in-water emulsified cosmetic of this embodiment may further contain an oil agent. For the purpose of the present specification, oil agents do not include the oil-soluble ultraviolet absorber (c) or the higher alcohol of 12 to 24 carbon atoms (g).

Examples of oil agents include hydrocarbon oils such as liquid paraffins, light liquid isoparaffins, dodecane, isododecane, tetradecane, isotetradecane, hexadecane, isohexadecane, squalane, vegetable squalane, vaseline, polyisobutylene and polybutene; ester oils such as isopropyl myristate, isopropyl palmitate, octyldodecyl myristate, glyceryl trioctanoate (triethylhexanoin), polyglyceryl diisostearate, propylene glycol di(caprylate/caprate), butylene glycol di(caprylate/caprate), dicaprylyl carbonate, diglyceryl triisostearate, glyceryl tribehenate, diisostearyl malate, neopentyl glycol dioctanoate and alkyl benzoate; higher fatty acids such as lauric acid, myristic acid, palmitic acid, stearic acid, behenic acid and oleic acid; and silicone oils such as polydimethylsiloxane (dimethicone), polymethylphenylsiloxane (diphenyldimethicone), phenyltrimethicone, diphenylsiloxyphenyltrimethicone, amino-modified silicone, epoxy-modified silicone, carboxy-modified silicone, polyether-modified silicone and alkyl-modified silicone and mixtures thereof. A single oil agent may be used alone, or two or more may be used in combination.

The oil-in-water emulsified cosmetic of this embodiment may also contain, in addition to these components, other additives such as pH regulators, chelating agents, antioxidants, antifading agents, antiseptic agents, drug and/or actives components, stabilizers, powders, pigments and perfumes, added as appropriate in order to impart various effects.

The oil-in-water emulsified cosmetic of this embodiment can be produced by the following steps, as a non limitative example.
1) The oil-soluble ultraviolet absorber (c), and if necessary the nonionic surfactant (f) and higher alcohol of 12 to 24 carbon atoms (g) and oil agent, are mixed and stirred while heating to obtain an oil-based mixture.
2) The aqueous phase (a), water-soluble ultraviolet absorber (d) and other components as necessary are mixed and stirred, to obtain an aqueous mixture.
3) The anionic surfactant (b), crosspolymer (e) and if necessary a thickening agent other than component (e) are added to the aqueous phase (a) and stirred while heating, and the oil-based mixture obtained in 1) is further added and the mixture is stirred while heating.
4) The aqueous mixture obtained in 2) and other components as necessary are added to the mixture obtained in 3), and the mixture is stirred while cooling.

Since the oil-in-water emulsified cosmetic of this embodiment gives a fresh and watery sensation during application and does not produce stickiness after application, it can be suitably used as a skin cosmetic, and is preferably used by application onto the skin (excluding the scalp), and more preferably onto the face, body and limbs. The oil-in-water emulsified cosmetic of this embodiment can be utilized as a lotion, a milky lotion, essence, cream or cosmetic base, for example. It can be used as skin care composition or a make-up composition. Moreover, the oil-in-water emulsified cosmetic of this embodiment is suitable as a sunscreen cosmetic because it has a high ultraviolet ray-protecting effect.
The oil-in-water emulsified cosmetic of the present invention is also used for preventing and/or reducing the effects of UV radiations onto the keratinic materials, in particular onto the skin, in particular for protecting the skin from sun-damage, sunburn, and/or photo-aging.

### Examples

The present invention will now be explained in specific detail through the following examples, with the understanding that the invention is not limited by these examples.

### (1) Preparation of oil-in-water emulsified cosmetics

Oil-in-water emulsified cosmetics with different components and their contents were prepared by the following method, based on the compositions listed in Table 1 (Examples 1 to 8 and Comparative Examples 1 to 6).

A mixed solution at 80°C comprising the oil-soluble ultraviolet absorber (c), nonionic surfactant (f), higher alcohol of 12 to 24 carbon atoms (g) and oil agent (mixture A), and a mixed solution at room temperature containing the aqueous phase (a) (water), water-soluble ultraviolet absorber (d), inorganic ultraviolet absorber and pH regulator (tromethamine) (mixture B), were prepared.

An aqueous phase (a) (water and phenoxyethanol), anionic surfactant (b), crosspolymer (e), thickening agent other than component (e) and a chelating agent (disodium EDTA) were mixed at 80°C using a stirrer, and then the prepared mixture A was added and the mixture was stirred. The obtained mixture was allowed to naturally cool, and the prepared mixture B was added and stirred with it at 60°C. The mixture was again allowed to naturally cool, and an aqueous dispersion containing an aqueous phase (a) (butylene glycol and propanediol) and methylene bis-benzotriazolyl tetramethylbutylphenol (h) was added and stirred with it at 50°C. The mixture was again allowed to naturally cool, and the other components were added and stirred with it at 40°C to obtain an oil-in-water emulsified cosmetic. The contents (units: mass%) of each of the materials were as shown in Table 1.

### (2) Stability evaluation

### (2-1: Storage for 1 month at 50°C)

Each of the prepared oil-in-water emulsified cosmetics was filled into a transparent container and sealed with a cap, and then stored for 1 month at 50°C. Any separation between the oil phase and aqueous phase after storage was observed. An evaluation of Y was assigned if no separation between the oil phase and aqueous phase was observed, and an evaluation of N was assigned if separation between the oil phase and aqueous phase was observed.

### (2-2: Cycle test at 40°C and -10°C)

Each of the prepared oil-in-water emulsified cosmetics was filled into a transparent container and sealed with a cap, and then storage at 40°C for 12 hours and storage at -10°C for 12 hours were alternately repeated for a total storage period of 1 month. An evaluation of Y was assigned if no separation between the oil phase and aqueous phase was observed, and an evaluation of N was assigned if separation between the oil phase and aqueous phase was observed.

### (3) Ultraviolet ray-protecting effect

Each of the prepared cosmetics was evenly coated onto a PMMA plate at a coating amount of 1.3 mg/cm², and dried. An SPF analyzer (UV-2000S by Labosphere) was then used to measure the *in vitro* SPF value, which was evaluated based on the following scale.

### (Evaluation scale)

A: *in vitro* SPF value of ≥50
B: *in vitro* SPF value of ≥30 and <50
C: *in vitro* SPF value of <30

### (4) Organoleptic evaluation

The fresh and watery sensation during application of each of the prepared cosmetics and their lack of stickiness after application were evaluated based on the following scale, in a single use test on skin by an evaluation panel of cosmetic experts of an organization to which the present inventors belong.

### (Fresh and watery sensation)

A: Strong
B: Moderate
C: Almost none
D: None

### (Lack of stickiness)

A: Absolutely no stickiness
B: Almost no stickiness
C: Slight stickiness
D: Stickiness

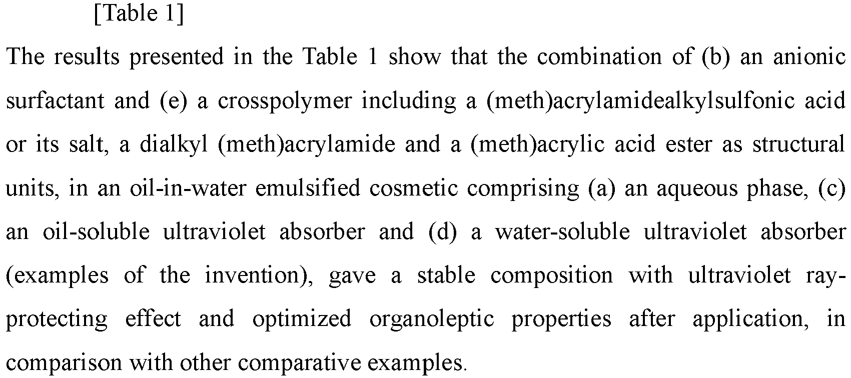

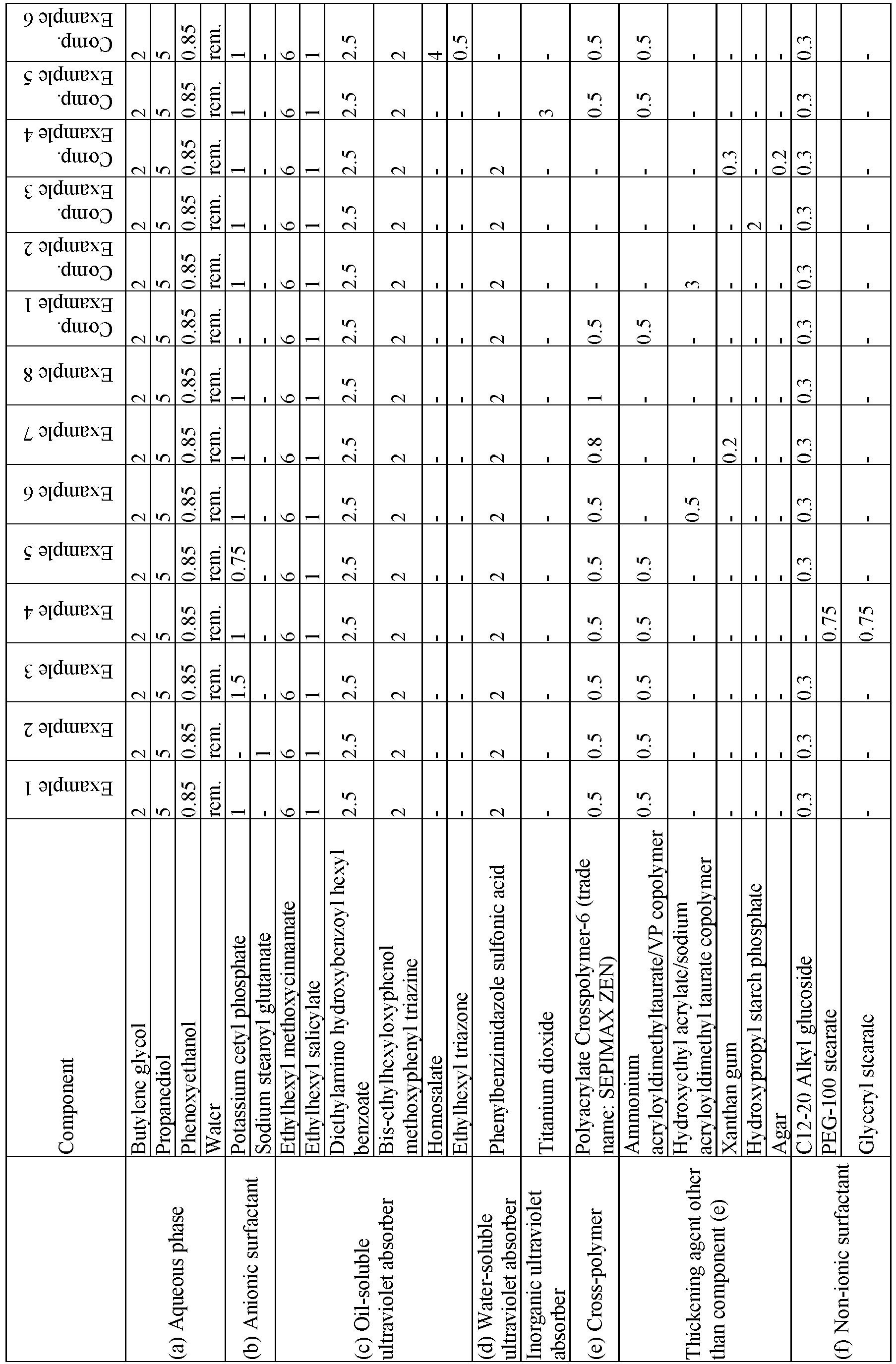

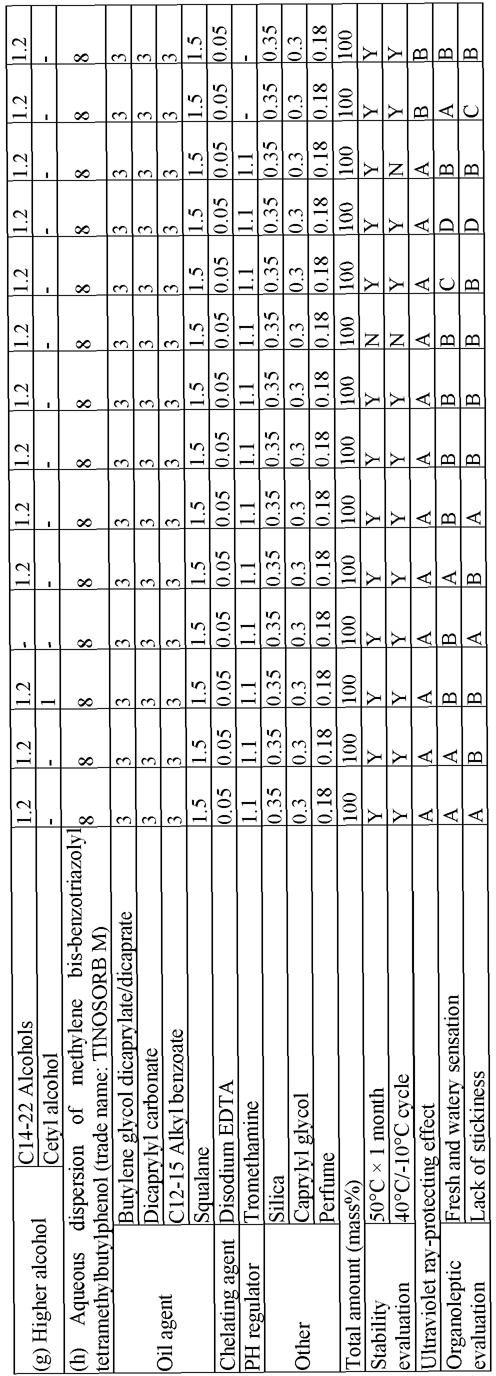

## Claims

1. An oil-in-water emulsified cosmetic comprising, in a cosmetically acceptable medium, (a) an aqueous phase, (b) an anionic surfactant, (c) an oil-soluble ultraviolet absorber, (d) a water-soluble ultraviolet absorber and (e) a crosspolymer including a (meth)acrylamidealkylsulfonic acid or its salt, a dialkyl (meth)acrylamide and a (meth)acrylic acid ester as structural units, wherein content of component (e) ranges from 0.01 to 1.5 mass% based on the total mass of the oil-in-water emulsified cosmetic.

2. The oil-in-water emulsified cosmetic according to claim 1, which further comprises at least one component selected from the group consisting of nonionic surfactants and higher alcohols of 12 to 24 carbon atoms.

3. The oil-in-water emulsified cosmetic according to claim 2, wherein the total content of the anionic surfactant, the nonionic surfactant and the higher alcohol of 12 to 24 carbon atoms is from 1 to 5 mass% based on the total mass of the oil-in-water emulsified cosmetic.

4. The oil-in-water emulsified cosmetic according to any one of claims 1 to 3, which further comprises methylene bis-benzotriazolyl tetramethylbutylphenol.

5. The oil-in-water emulsified cosmetic according to any one of claims 1 to 4, which is a sunscreen cosmetic composition.

6. The oil-in-water emulsified cosmetic according to anyone of claims 1 to 5, for use for preventing and/or reducing the effects of UV radiations onto the keratinic materials, in particular onto the skin, in particular protecting the skin from sun-damage, sunburn, and/or photo-aging.

## Patentansprüche

1. Kosmetische Öl-in-Wasser-Emulsion, umfassend, in einem kosmetisch annehmbaren Medium, (a) eine wässrige Phase, (b) ein anionisches Tensid, (c) einen öllöslichen UV-Absorber, (d) einen wasserlöslichen UV-Absorber und (e) ein Kreuzpolymer, das eine (Meth)Acrylamid-Alkylsulfonsäure oder ihr Salz, ein Dialkyl(meth)acrylamid und ein (Meth)Acrylsäureester als Struktureinheiten umfasst, wobei der Gehalt der Komponente (e) in einem Bereich von 0,01 bis 1,5 Ma%, bezogen auf die Gesamtmasse der kosmetischen Öl-in-Wasser-Emulsion, liegt.

2. Kosmetische Öl-in-Wasser-Emulsion nach Anspruch 1, die ferner mindestens eine Komponente umfasst, die ausgewählt ist aus der Gruppe, die besteht aus nichtionischen Tensiden und höheren Alkoholen mit 12 bis 24 Kohlenstoffatomen.

3. Kosmetische Öl-in-Wasser-Emulsion nach Anspruch 2, wobei der Gesamtgehalt des anionischen Tensids, des nichtionischen Tensids und des höheren Alkohols mit 12 bis 24 Kohlenstoffatomen zwischen 1 und 5 Ma%, bezogen auf die Gesamtmasse der kosmetischen Öl-in-Wasser-Emulsion, beträgt.

4. Kosmetische Öl-in-Wasser-Emulsion nach einem der Ansprüche 1 bis 3, die ferner Methylen-Bis-Benzotriazolyl-Tetramethylbutylphenol umfasst.

5. Kosmetische Öl-in-Wasser-Emulsion nach einem der Ansprüche 1 bis 4, die eine kosmetische Sonnenschutzmittelzusammensetzung ist.

6. Kosmetische Öl-in-Wasser-Emulsion nach einem der Ansprüche 1 bis 5 zur Verwendung bei der Prävention und/oder Verminderung der Wirkungen von UV-Strahlungen auf die Keratinmaterialien, insbesondere auf der Haut, die insbesondere die Haut vor Sonnenschäden, Sonnenbrand und/oder Lichtalterung schützt.

## Revendications

1. Emulsion cosmétique huile-dans-eau comprenant, dans un milieu cosmétiquement acceptable, (a) une phase aqueuse, (b) un tensioactif anionique, (c) un absorbeur d'ultraviolets soluble dans l'huile, (d) un absorbeur d'ultraviolets soluble dans l'eau et (e) un polymère croisé comprenant un acide (méth)acrylamidealkylsulfonique ou son sel, un (méth)acrylamide de dialkyle et un ester d'acide (méth)acrylique en tant qu'unités structurelles, dans lequel la teneur du composant (e) varie de 0.01 à 1,5 % en masse par rapport à la masse totale du cosmétique émulsionné huile dans eau.

2. Emulsion cosmétique huile-dans-eau selon la revendication 1, qui comprend en outre au moins un composant choisi dans le groupe constitué par les tensioactifs non ioniques et les alcools supérieurs de 12 à 24 atomes de carbone.

3. Emulsion cosmétique huile-dans-eau selon la revendication 2, dans laquelle la teneur totale du tensioactif anionique, du tensioactif non ionique et de l'alcool supérieur de 12 à 24 atomes de carbone est de 1 à 5 % en masse par rapport à la masse totale de l'émulsion cosmétique huile-dans-eau.

4. Emulsion cosmétique huile-dans-eau selon l'une quelconque des revendications 1 à 3, qui comprend en outre du méthylène bis-benzotriazolyl tétraméthylbutylphénol.

5. Emulsion cosmétique huile-dans-eau selon l'une des revendications 1 à 4, qui est une composition cosmétique de protection solaire.

6. Emulsion cosmétique huile-dans-eau selon l'une des revendications 1 à 5, destinée à être utilisée pour prévenir et/ou réduire les effets des radiations UV sur les matières kératiniques, en particulier sur la peau, notamment pour protéger la peau des dommages causés par le soleil, des coups de soleil et/ou du photovieillissement.
